# FASCICULE DE BREVET EUROPEEN

(11) **EP 2 809 697 B1**
(45) Date de publication et mention de la délivrance du brevet: **19.07.2017**
(21) Numéro de dépôt: 13701788.5
(22) Date de dépôt: 30.01.2013
(51) Int. Cl.: C08F 293/00, C08F 26/06, C08F 14/00, C08F 18/04, A61K 8/81, A61K 8/06, C08F 2/20, C08F 2/22, C08F 214/06, C08F 2/38, C08F 214/18, C08F 218/08, C08F 226/10, C09K 8/588, C09D 127/08, C09D 131/04, C09J 127/08, C09J 131/04

(54) **STABILISANTS RÉACTIFS POLY(N-VINYL LACTAME) VIVANTS POUR POLYMÉRISATION EN PHASE DISPERSÉE**
LEBENDE POLY-(N-VINYL LACTAM-)REAKTIVE STABILISATOREN FÜR EINE POLYMERISIERUNG DISPERGIERTER PHASEN
LIVE POLY(N-VINYL LACTAM) REACTIVE STABILISERS FOR DISPERSED PHASE POLYMERISATION

(30) Priorité: 31.01.2012 FR 1200291; 18.10.2012 FR 1202795
(43) Date de publication de la demande: 10.12.2014
(73) Titulaire: Rhodia Operations, 75009 Paris (FR)
(72) Inventeur: DESTARAC, Mathias, F-31100 Toulouse (FR); WILSON, James, F-60580 Coye La Foret (FR)
(74) Mandataire: Cordier, Pascal Christian
(86) Numéro de dépôt international: PCT/EP2013/051788
(87) Numéro de publication internationale: WO 2013/113750

(56) Documents cités:
- EP-A1- 1 510 533
- EP-A1- 1 514 884
- WO-A1-2010/083569
- FR-A1- 2 965 264
- CHIN-ICHI YUSA ET AL: "Thermo-Responsive Diblock Copolymers of Poly(N-isopropylacrylamide) and Poly(N-vinyl-2-pyrroridone) Synthesized via Organotellurium-Mediated Controlled Radical Polymerization (TERP)", MACROMOLECULES, AMERICAN CHEMICAL SOCIETY, WASHINGTON, DC; US, vol. 40, 7 août 2007 (2007-08-07), pages 5907-5915, XP002631832, ISSN: 0024-9297, DOI: 10.1021/MA070769X [extrait le 2007-07-10]

## Description

La présente invention a trait au domaine des polymérisations mettant en oeuvre des monomères au sein d'une phase liquide dispersée dans une phase liquide continue (polymérisation en phase dispersée), incluant notamment les polymérisations en émulsion. Plus précisément, l'invention concerne des polymérisations de ce type mettant en oeuvre des agents de stabilisation de la phase dispersée particuliers, dits « stabilisants réactifs ».

On connaît diverses réactions de polymérisation mettant en oeuvre des monomères à l'état de gouttelettes dispersées dans une phase continue (généralement une phase aqueuse) avec des agents de stabilisation de type stabilisants réactifs.

De façon générale, le stabilisant réactif mis en oeuvre dans ces réactions est une molécule qui permet d'une part de stabiliser les gouttelettes de la phase dispersée contenant les monomères et qui est en outre propre à être engagé dans la réaction de polymérisation. De tels stabilisants réactifs présentent, entre autres, l'avantage de se substituer en tout ou partie aux tensioactifs additionnels qu'il est sinon nécessaire de mettre en oeuvre pour stabiliser la phase dispersée et ils conduisent de ce fait à des dispersions de polymères (latex) intéressants en ce sens qu'ils sont exempts de tels tensioactifs ou tout au moins en ce qu'ils présentent une teneur réduite en de tels réactifs.

De tels stabilisants réactifs ont notamment été proposés pour réaliser des polymérisations en émulsion de monomères hydrophobes de type vinylique, qui sont des molécules à caractère hydrophile ou amphiphile comprenant typiquement une chaîne polymère hydrophile et un groupement réactif propre à induire une réaction de polymérisation radicalaire contrôlée des monomères, par exemple de type ATRP (atom transfer radical polymerization) ou NMP (nitroxide-mediated polymerization), ou RAFT ou MADIX en présence de radicaux libres, avec par exemple un groupe de type xanthate (porteur de fonctions -S(C=S)O-) en extrémité de chaîne. Ces stabilisants réactifs assurent d'une part la stabilisation de l'émulsion des gouttelettes de la phase dispersée du fait de leur caractère hydrophile, et ils assurent d'autre part le rôle d'agent de contrôle de la polymérisation, typiquement par des processus de transfert réversible par addition-fragmentation, ce par quoi les chaînes polymères croissent à partir de cet agent de contrôle par consommation progressive des monomères contenus dans chaque un caractère dit « vivant », en ce sens que chaque étape d'incorporation d'une unité monomère dans la chaîne conduit à un polymère qui reste porteur d'une extrémité de chaîne réactivable pour l'incorporation ultérieure d'une autre unité monomère selon le même processus d'addition-fragmentation (pour plus de détail concernant la polymérisation radicalaire contrôlée ou « vivante » et l'obtention de chaînes croissant à partir de l'agent de transfert, on pourra notamment se reporter à Handbook of RAFT polymérisation, Ed Barner-Kowollik C. Wiley-VCH 2008 ou bien encore aux procédés décrits dans WO96/30421, WO 98/01478, WO 99/35178, WO 98/58974, WO 00/75207 et WO 01/42312, WO 99/35177 ,WO 99/31144, FR2794464 ou WO 02/26836).

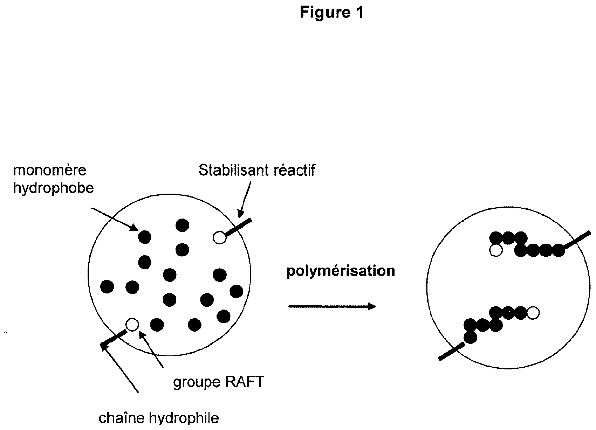

Avec les stabilisants réactifs du type précité, qui induisent une polymérisation radicalaire contrôlée, on obtient la formation des chaînes polymères vivantes au sein des gouttelettes dispersées, avec une incorporation de l'agent stabilisant au sein de ces chaînes polymère et ce dès le début de la polymérisation, mais en conservant néanmoins une stabilisation de l'émulsion tout au long de la polymérisation sans nécessairement avoir à employer de tensioactif additionnel, la chaîne hydrophile restant schématiquement en permanence à la périphérie des gouttelettes, comme illustré sur la Figure 1 ci-annexée.

Des stabilisants réactifs du type précité ont notamment été proposés dans les procédés décrits dans les publications suivantes :
**Nitroxide-Mediated Controlled/Living Free-Radical Surfactant-Free Emulsion Polymerization of Methyl Methacrylate using a Poly(Methacrylic Acid)-based Macroalkoxyamine Initiator.**
   C. Dire, S. Magnet, L. Couvreur, B. Charleux Macromolecules 42(1), 95-103 (2009)
**PEO-based Block Copolymers and Homopolymers as Reactive Surfactants for AGET ATRP of Butyl Acrylate in Miniemulsion.**
   W. Li, K. Min, K. Matyjaszewski, F. Stoffelbach, B. Charleux Macromolecules 41, 6387-6392 (2008)
**Effective ab Initio Emulsion Polymerization under RAFT Control**
   Christopher J. Ferguson, Robert J. Hughes, Binh T. T. Pham, Brian S. Hawkett, Robert G. Gilbert, Algirdas K. Serelis, and Christopher H. Such Macromolecules (2002)
**Surfactant-free controlled/living radical emulsion (co)polymerization of n-butyl acrylate and methyl methacrylate via RAFT using amphiphilic poly(ethylene oxide)-based trithiocarbonate chain transfer agents.**
   J. Rieger, G. Osterwinter, C. Bui, F. Stoffelbach, B. Charleux Macromolecules (2009)

Bien qu'intéressants dans l'absolu, une des limitations des stabilisants réactifs décrits jusqu'à présent est qu'ils sont adaptés uniquement à la polymérisation de certains monomères vinyliques, essentiellement de type méthacrylate et acrylate d'alkyle.

Un but de la présente invention est de fournir un procédé de polymérisation en phase dispersée adapté à la mise en oeuvre d'un grand nombre de monomères hydrophobes et notamment adapté à la polymérisation en phase dispersée d'esters de vinyle et de monomères N-vinyliques hydrophobes

A cet effet, la présente invention propose la mise en oeuvre d'un nouveau type de stabilisant réactif, à savoir une chaîne de polymère vivante issue d'une polymérisation radicalaire contrôlée RAFT ou MADIX de monomères N-(vinyl lactame).

Plus précisément, la présente invention a pour objet un procédé de préparation d'un polymère comprenant une étape (E1) de polymérisation en phase dispersée en présence d'un stabilisant réactif, dans laquelle on met en présence au sein d'une phase aqueuse :
- au moins un monomère éthyléniquement insaturé, en général à l'état dispersé ;
- au moins une source de radicaux libres ; et
- un stabilisant réactif comprenant une chaîne polymère incluant (voire consistant en) des unités monomères N-(vinyl lactame) et un groupe thiocarbonylthio -S(C=S)-,
ladite étape (E1) étant de préférence conduite à une température inférieure à la température de dégradation du groupe thiocarbonylthio -S(C=S)- du stabilisant réactif, de préférence à une température inférieure à 40°C, par exemple à une température inférieure ou égale à 30°C (le procédé de l'invention pouvant par exemple être avantageusement conduit à température ambiante.

La source de radicaux libres employée dans l'étape (E1) est de préférence un initiateur de polymérisation de type redox. Plus généralement, il peut s'agir de tout initiateur capable de générer des radicaux libres dans les conditions de l'étape (E1), de préférence à une température inférieure à 40°C, plus avantageusement à une température inférieure ou égale à 30°C.

La polymérisation en phase dispersée réalisée dans le cadre de l'invention est, en général, une polymérisation qui conduit, en fin de réaction à une émulsion ou une dispersion du polymère synthétisé dans la phase aqueuse.

Pour ce faire, selon un premier mode de réalisation, les monomères peuvent typiquement être mis en oeuvre à l'état dispersé au sein de la phase aqueuse (polymérisation en émulsion, par exemple).

Alternativement, les monomères peuvent être employés sans que ne se forme de dispersion de monomère dans la phase aqueuse. Dans ce cas, le polymère précipite progressivement et se disperse sous forme des particules (polymérisation en dispersion, typiquement). Par exemple, les monomères peuvent être introduits, de façon progressive au cours de la polymérisation en conservant en permanence une concentration en lesdits monomères inférieures à leur limite de solubilité.

Selon un mode de réalisation particulier, on peut introduire les monomères de façon continue ou semi-continue, dans une phase aqueuse contenant initialement une partie seulement des monomères à une concentration initiale inférieure à la limite de solubilité.

Dans tous les cas, le stabilisant réactif utilisé selon l'invention peut stabiliser l'émulsion ou la dispersion de polymère obtenu en fin de réaction. Dans certains modes de réalisation (polymérisation en émulsion, notamment), il peut aussi stabiliser la dispersion initiale de monomères.

Le stabilisant réactif employé dans l'étape (E1), est typiquement d'un polymère issu d'une étape (E0) de polymérisation radicalaire contrôlée d'une composition comprenant :
- des monomères contenant (et le plus souvent consistant en) des monomères N-vinyl lactame, identiques ou différents (et généralement identiques);
- un agent de contrôle de la polymérisation radicalaire, par exemple comprenant un groupe thiocarbonylthio -S(C=S)- ; et
- un initiateur de la polymérisation radicalaire qui est typiquement un système redox.

Selon un mode de réalisation particulier de l'invention, l'initiateur de la polymérisation radicalaire employé peut être un système thermique.

Les travaux qui ont été réalisés par les inventeurs à ce sujet ont maintenant permis de mettre en évidence qu'il s'avère possible de réaliser une polymérisation radicalaire d'unités monomères N-vinyl lactame à la fois en milieu aqueux et de façon efficacement contrôlée, sous réserve d'amorcer cette réaction radicalaire par le biais d'un système redox.

Selon un autre mode de réalisation, le stabilisant réactif employé dans l'étape (E1) est préparé en milieu solvant organique ou sans solvant. Le cas échéant, il est replacé dans l'eau préalablement à l'étape (E1).

Les conditions de l'étape (E1) de l'invention sont adaptées à la polymérisation d'un grand nombre de monomères éthyléniquement insaturés, incluant entre autres les esters vinyliques et d'autres monomères N-vinyliques hydrophobes. L'invention donne accès à d'autres polymérisations, et elle permet notamment d'envisager la polymérisation des monomères halogénés décrits plus en détails ci-après dans la présente description.

A l'issue de l'étape (E1), on obtient généralement des dispersions de polymères (latex) stables, avec des particules de polymères qui ont une masse et une taille contrôlées, ce qui constitue un autre objet particulier de la présente invention. L'invention peut en particulier donner accès à des latex de polyoléfines fluorées (notamment polymères vinyliques fluorés) exempts de tensioactifs fluorés ou tout au moins présentant un taux de tensioactif bien inférieurs à ceux communément employés.

Alternativement, si on met en oeuvre une faible quantité de monomères lors de l'étape (E1), cette étape peut conduire à la formation des polymères vivants comprenant un premier bloc à base de monomères N-vinyl lactame sur lequel est greffé un petit bloc à base d'unités hydrophobes terminé par un groupe thiocarbonylthio -S(C=S)- (ces polymères vivants étant désignés ci-après par le terme de « stabilisants réactifs greffés »). Dans ce cas, on forme généralement à l'issue de l'étape (E1), un milieu aqueux comprenant ces stabilisants réactifs greffés regroupés en structures de type micelles qui sont notamment utilisables à titre de site de polymérisation pour des polymérisations radicalaires contrôlées ultérieures de monomères éthyléniquement insaturés, généralement hydrophobes.

Différentes caractéristiques et modes de réalisation avantageux du procédé de l'invention vont maintenant être décrits plus en détails.

### Le stabilisant réactif

Les stabilisants réactifs employés dans l'étape (E1) peuvent être employés seuls ou en association avec d'autres stabilisants. Ainsi, selon un mode de réalisation intéressant, l'étape (E1) est conduite en l'absence d'autres agents de stabilisation, notamment en l'absence d'agents tensioactifs. Néanmoins, selon un autre mode de réalisation envisageable, l'étape (E1) peut être conduite en mettant en oeuvre les agents stabilisants réactifs de l'invention avec d'autres co-stabilisants, par exemples des tensioactifs.

Le stabilisant réactif employé dans l'étape (E1) peut typiquement être employé à une concentration massique allant de 0,01% à 50%.

Le ratio massique polymère/stabilisant qui reste généralement supérieur à 0,005, de préférence supérieur à 0,01, par exemple supérieur à 0,02, notamment pour assurer un effet de stabilisation suffisant. Ce ratio massique polymère/stabilisant n'a généralement pas besoin d'être supérieur à 400, et il est typiquement inférieur ou égal à 100, voire inférieur ou égal à 50. Ainsi, ce ratio peut notamment être compris entre 0,005 et 400, par exemple 0,05 et 300, notamment entre 0,02 et 50.

### Les unités monomères N-vinyl lactame du stabilisant réactif

Les monomères constitutifs du stabilisant réactif employé dans l'étape (E1), et employés le cas échéant dans l'étape (E0), sont des monomères éthyléniquement insaturés répondant en général à la formule suivante : 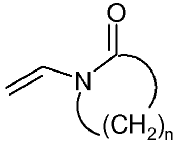 où n est un entier allant de 2 à 6, typiquement égal à 3 (N-vinyl pyrrolidone), 4 (N-vinyl pipéridone) ou 5 (N-vinyl caprolactame).

De préférence, les unités monomères comprises dans le stabilisant réactif employé dans l'étape (E1) comprennent de la N-vinyl pyrrolidone (NVP). Selon un mode de réalisation particulier, l'ensemble des monomères employés dans l'étape (E) sont des monomères NVP. D'autres monomères N-vinyl lactame peuvent également s'avérer avantageux selon l'invention, parmi lesquels on peut notamment citer, de façon non limitative, la N-vinylcaprolactame (NVCL).

Les unités monomères présentes dans le stabilisant réactif employé dans l'étape (E1) peuvent par exemple consister exclusivement en des unités monomères NVP, en des unités monomères NVCL ou bien en un mélange d'unités NVP ou NVCL (réparties sous forme de blocs, sous forme statistique ou sous forme de gradient).

Alternativement, selon un mode de réalisation possible, les monomères N-vinyl lactame peuvent comprendre d'autres unités monomères que des N-vinyl lactame, typiquement introduites dans l'étape (E0) en copolymérisant les monomères N-vinyl lactame avec des monomères éthyléniquement insaturés non N-vinyl lactame, ce qui conduit typiquement à la formation d'un bloc polymère statistique ou à gradient lors de l'étape (E0). Lorsque d'autres unités monomères que des N-vinyl lactame sont présentes dans (E0), la teneur en monomères N-vinyl lactame reste généralement supérieure ou égale à 50%, plus préférentiellement supérieure ou égale à 70% (par exemple d'au moins 80%, voire d'au moins 90%), en masse par rapport à la masse totale de monomères mis en oeuvre dans l'étape (E0).

Parmi les monomères éthyléniquement insaturés non N-vinyl lactame qu'il peut être intéressant de copolymériser avec les monomères N-vinyl lactame lors de l'étape (E0), on peut notamment citer la N-vinylimidazole (1-vinylimidazole), vinyl carbazole (N-vinyl carbazole), vinyl phthalimide (N-vinyl phthalimide), vinyl acetamide (N-vinyl acetamide), N-methyl N-vinylacetamide, vinyl formamide (N-vinyl formamide), les esters vinyliques. D'autres monomères non N-vinyl lactame peuvent être employés, parmi lesquels on peut citer, de façon non limitative, l'acide acrylique, l'acrylamide, le 2-acrylamido-2-methylpropanesulfonic acid (l'AMPS), l'acrylamido propyl trimethyl ammonium chloride (l'APTAC), le N,N'dimethyl acrylamide, le N-isopropylacrlamide (NIPAM), le N,N-Diethyl acrylamide, le diallyl dimethyl ammonium chloride (le DADMAC), l'acide vinylphosphonique, les phosphonates dialkylvinyliques, ou bien encore le vinyl sulfonate.

### Le groupe thiocarbonylthio du stabilisant réactif

Ce groupe est typiquement introduit via l'agent de contrôle employé dans la polymérisation radicalaire contrôlée mise en oeuvre dans l'étape (E0), qui est typiquement un agent de contrôle RAFT ou MADIX. Selon un mode de réalisation particulier, cet agent de contrôle employé dans l'étape (E0) peut être porteur de plusieurs groupes thiocarbonylthio.

Le groupe thiocarbonylthio présent sur le stabilisant réactif répond typiquement à la formule -S(C=S)-Z où Z est tel que fini ci-dessous, ce groupe étant typiquement obtenu en mettant en oeuvre dans l'étape (E0) un agent de contrôle qui répond à la formule (A) ci-dessous : 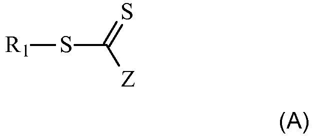 où :
- Z représente :
   - un atome d'hydrogène,
   - un atome de chlore,
   - un radical alkyl éventuellement substitué, aryl éventuellement substitué,
   - un hétérocycle éventuellement substitué,
   - un radical alkylthio éventuellement substitué,
   - un radical arylthio éventuellement substitué,
   - un radical alkoxy éventuellement substitué,
   - un radical aryloxy éventuellement substitué,
   - un radical amino éventuellement substitué,
   - un radical hydrazine éventuellement substitué,
   - un radical alkoxycarbonyl éventuellement substitué,
   - un radical aryloxycarbonyl éventuellement substitué,
   - un radical carboxy, acyloxy éventuellement substitué,
   - un radical aroyloxy éventuellement substitué,
   - un radical carbamoyle éventuellement substitué,
   - un radical cyano,
   - un radical dialkyl- ou diaryl-phosphonato,
   - un radical dialkyl-phosphinato ou diaryl-phosphinato,
      ou
   - une chaîne polymère
   et
- R₁ représente :
   - un groupe alkyle, acyle, aryle, aralkyle, alcène ou alcyne éventuellement substitué,
   - un cycle carboné ou un hétérocycle, saturé ou non, aromatique éventuellement substitué, ou
   - une chaîne polymère.

Les groupes R₁ ou Z, lorsqu'ils sont substitués, peuvent l'être par des groupes phényles éventuellement substitués, des groupes aromatiques éventuellement substitués, des cycles carbonés saturés ou non, des hétérocycles saturé ou non, ou des groupes : alkoxycarbonyle ou aryloxycarbonyle (-COOR), carboxy (-COOH), acyloxy (-O₂CR), carbamoyle (-CONR₂), cyano (-CN), alkylcarbonyle, alkylarylcarbonyle, arylcarbonyle, arylalkylcarbonyle, phtalimido, maleïmido, succinimido, amidino, guanidimo, hydroxy (-OH), amino (-NR₂), halogène, perfluoroalkyle CₙF₂ₙ₊₁, allyle, époxy, alkoxy (-OR), S-alkyle, S-aryle, des groupes présentant un caractère hydrophile ou ionique tels que les sels alcalins d'acides carboxyliques, les sels alcalins d'acide sulfonique, les chaînes polyoxyde d'alkylène (PEO, POP), les substituants cationiques (sels d'ammonium quaternaires), R représentant un groupe alkyle ou aryle, ou une chaîne polymère.

Selon un mode de réalisation particulier, R₁ est un groupe alkyle substitué ou non, de préférence substitué.

Les groupes alkyle, acyle, aryle, aralkyle ou alcyne éventuellement substitués auxquels il est fait référence dans la présente description présentent généralement 1 à 20 atomes de carbone, de préférence 1 à 12, et plus préférentiellement 1 à 9 atomes de carbone. Ils peuvent être linéaires ou ramifiés. Ils peuvent être également substitués par des atomes d'oxygène, sous forme notamment d'esters, des atomes de soufre ou d'azote.

Parmi les radicaux alkyle, on peut notamment citer le radical méthyle, éthyle, propyle, butyle, pentyle, isopropyle, tert-butyle, pentyle, hexyle, octyle, decyle ou dodécyle.

Les groupes alcynes au sens de la présente description sont des radicaux généralement de 2 à 10 atomes de carbone, ils présentent au moins une insaturation acétylénique, tel que le radical acétylenyle.

Les groupes acyle au sens de la présente description sont des radicaux présentant généralement de 1 à 20 atomes de carbone avec un groupement carbonyle.

Parmi les radicaux aryle utilisable selon l'invention, on peut notamment citer le radical phényle, éventuellement substitué notamment par une fonction nitro ou hydroxyle.

Parmi les radicaux aralkyle, on peut notamment citer le radical benzyle ou phénéthyle, éventuellement substitué notamment par une fonction nitro ou hydroxyle.

Lorsque R₁ ou Z est une chaîne polymère, cette chaîne polymère peut être issue d'une polymérisation radicalaire ou ionique ou issue d'une polycondensation.

Dans le cadre de la présente invention, il est notamment intéressant d'employer à titre d'agents de contrôle des xanthates, des dithiocarbamates, ou des dithiocarbazates.

Avantageusement, on utilise, comme agent de contrôle dans l'étape (E0), des composés xanthates, comme par exemple le O-ethyl-S-(1-methoxycarbonyl ethyl) xanthate de formule (CH₃CH(CO₂CH₃))S(C=S)OCH₂CH₃, et, plus généralemement, le stabilisant réactif de l'étape (E1) est de préférence porteur de tels groupes.

Un agent de contrôle bien adapté à la mise en oeuvre de l'étape (E0) est le composé commercialisé par la société Rhodia sous le nom de Rhodixan A1.

### Les sources de radicaux libres utilisables dans les étape (E0) et (E1)

Les sources de radicaux libres employés dans les étapes (E0) et (E1) peuvent être identiques ou différentes, et elles sont typiquement identiques pour des raisons pratiques.

Une source de radicaux libre adaptée aussi bien dans l'étape (E0) que dans l'étape (E1) est un initiateur de polymérisation de type Redox qui comprend deux agents, à savoir un agent oxydant et un agent réducteur, qui peuvent être introduits simultanément ou bien consécutivement.

Selon un mode de réalisation intéressant, les agents réducteur et oxydant sont introduit de façon séparée, ce qui permet de retarder l'amorçage de la polymérisation jusqu'à l'introduction du second agent. Avantageusement, une étape employant un tel agent redox est conduite (i) en formant d'abord un mélange comprenant l'un des agents oxydant ou réducteur en mélange avec les monomères et l'agent de contrôle (qui est l'agent de transfert réactif dans l'étape (E1)), puis (ii) en ajoutant à ce mélange l'autre agent (respectivement réducteur ou oxydant).

Les effets avantageux mis en évidence par les inventeurs dans le cadre de la présente invention sont, en général, d'autant plus marqués que la différence entre les potentiels redox standards de l'oxydant et du réducteur (Eₒₓ-E_{red}) est importante. Il est préconisé dans ce cadre de l'invention que la différence entre les potentiels rédox standards de l'agent oxydant et de l'agent réducteur (Eₒₓ-E_{red}) soit comprise entre 1 et 2 V. Par ailleurs, notamment pour éviter des réactions d'oxydation des monomères N-vinyl lactame, il peut être intéressant que le potentiel redox standard d'oxydation Eₒₓ de l'agent oxydant (Ox) mis en oeuvre dans l'étape (E0) soit inférieur (de préférence d'au moins 0,2V, plus préférentiellement d'au moins 0,5 V, voire d'au moins 1V) à celui des monomères N-vinyl lactame employés. Plus généralement, il est préférable que le potentiel redox standard d'oxydation Eₒₓ de l'agent oxydant (Ox) soit suffisamment faible pour ne pas oxyder les monomères N-vinyl lactame. Les monomères NVP sont tout particulièrement sensibles à l'oxydation et il est préférable, lorsqu'on polymérise du NVP, que le potentiel d'oxydation Eₒₓ de l'agent oxydant soit inférieur à 2 V, plus préférentiellement inférieur à 1,8 V, par exemple entre 1,5 et 1,8 V.

Des agents oxydant particulièrement bien adaptés dans ce cadre sont les hydroperoxydes, et notamment l'hydroperoxyde de tertbutyle (t-BuOOH), qui est notamment intéressant lorsqu'on polymérise des monomères NVP. Le peroxyde d'hydrogène est un autre agent oxydant possible.

D'autre part, notamment lorsque les monomères employés sont des monomères de type NVP, il est préférable que les agents présents dans le système redox ne contiennent pas d'acides de nature à induire des réactions parasites des monomères propres à conduire à des sous produits non recherché, et plus généralement qu'ils ne contiennent pas de composés ayant un pKa suffisamment faible pour induire de telles réactions. Ainsi, de préférence, il est notamment préconisé d'employer des agents réducteur (Red) et oxydant (Ox) ayant un pKa supérieur à 4, plus préférentiellement supérieur à 6, voire à 6,5, et de préférence d'au moins 7, ce qui permet de réduire le taux de sous-produits, généralement à tout au plus quelques % dans le polymère synthétisé. Dans ce cadre, un agent réducteur particulièrement adapté est le sulfite de sodium (pKa=7,2), qui permet par exemple de limiter le taux de sous produits en deçà de 5% lors de la polymérisation de la NVP.

Des systèmes redox bien adaptés à la mise en oeuvre des étapes (E0) et (E1) du procédé de l'invention comprennent de l'hydroperoxyde de tertbutyle (t-BuOOH) à titre d'agent oxydant, associé à un agent réducteur choisi parmi l'acide ascorbique ou le sulfite de sodium.

Le système redox hydroperoxyde de tertbutyle/sulfite de sodium s'avère tout particulièrement intéressant, notamment lorsque les monomères employés dans l'étape (E0) sont ou comprennent des monomères NVP. L'emploi de ce système dans l'étape (E0) permet de polymériser de la NVP à température ambiante et dans l'eau avec un très faible taux de sous-produits, restant typiquement bien inférieur à 5%.

### Les conditions de mise en oeuvre des étapes (E0) et (E1)

Compte tenu de la mise en oeuvre du système redox dans l'étape (E0) cette étape est avantageusement conduite en milieu aqueux, typiquement en utilisant de l'eau à titre d'unique solvant. Elle permet ainsi d'obtenir un polymère directement en milieu aqueux sans avoir à utiliser de solvants organiques, ce qui rend le procédé particulièrement adapté à une utilisation à l'échelle industrielle.

Par ailleurs, les étapes (E0) et (E1) sont avantageusement mises en oeuvre à basse température, de préférence en dessous de 40°C, plus avantageusement à une température inférieure ou égale à 30°C, notamment entre 5 et 25°C. Ces deux étapes peuvent donc être réalisées par exemple à température ambiante, ce qui est un autre avantage du procédé de l'invention, en termes de coûts énergétiques.

La possibilité de conduire l'étape (E0) à faible température permet par ailleurs d'envisager sa mise en oeuvre pour la polymérisation de la N-vinylcaprolactame (NVCL) en milieu aqueux (dans l'eau ou, avantageusement, dans un mélange d'eau et d'un solvant hydrosoluble), qui nécessite une polymérisation à une température inférieure à son point de trouble (« cloud point » en anglais) qui est de 32°C. Dans ce cadre, le procédé de l'invention peut notamment être mis en oeuvre pour la synthèse de poly(N-vinylcaprolactame) ou de polymères à base à la fois d'unités monomères NVCL et NVP, amphiphiles ou non. Ces stabilisants réactifs à base de NVCL et NVP peuvent également être synthétisés en milieu non aqueux, puis utilisés dans l'eau par la suite pour (E1) après purification.

Les blocs polymères préparés dans les étapes (E0) et (E1) peuvent être des homopolymères ou bien des copolymères statistiques (ou à gradient, en particulier dans l'étape (E0)).

### Les monomères utilisables dans (E1)

Il s'agit généralement de monomères hydrophobes.

Le procédé de l'invention, notamment du fait du choix judicieux d'un stabilisant réactif particulier s'avère modulaire et peut être employé pour réaliser des polymérisations en phase dispersée (notamment des polymérisations en émulsion) d'un très grand nombre de monomères hydrophobes éthyléniquement insaturés dans l'étape (E1).

L'étape (E1) présente en particulier l'avantage de permettre la polymérisation en phase dispersée de monomères de type esters de vinyle. Ainsi, selon un premier mode de réalisation intéressant, les monomères hydrophobes utilisables dans l'étape (E1) peuvent comprendre des monomères esters de vinyle de formule CH2=CH-O-(C=O)-Rₐ, où Rₐ représente :
- un groupe alkyle, acyle, aryle, aralkyle, alcène ou alcyne éventuellement substitué, de préférence un groupe alkyle
- un cycle carboné ou un hétérocycle, saturé ou non, aromatique éventuellement substitué.

Rₐ peut notamment être un groupe alkyle, linéaire ou ramifié, comportant typiquement de 1 à 20, par exemple de 4 à 18 atomes de carbone, ces groupes alkyles pouvant éventuellement être halogénés (fluorés et ou chlorés) en tout ou partie.

Ainsi, l'étape (E1) s'avère par exemple tout particulièrement bien adaptée à la polymérisation en phase dispersée d'esters de vinyle choisis parmi l'acétate de vinyle, le pivalate de vinyle, le butyrate de vinyle, le trifluoroacétate de vinyle, le stéarate de vinyle, ou bien encore le néodécanoate de vinyle, ainsi que les mélanges de ces monomères, entre eux, ou avec d'autres monomères éthyléniquement insaturés, de type ester de vinyle ou non.

A noter à ce sujet que les stabilisants réactifs de l'invention se révèlent adaptés à une polymérisation en émulsion d'esters de vinyle, par exemple, en mode batch, notamment compte tenu de leur constante de transfert vis-à-vis des esters de vinyle. Selon un autre mode de réalisation, les monomères hydrophobes l'étape (E1) peuvent comprendre des composés vinyliques halogénés, tels que des chlorures ou fluorure de vinyle ou de vinylidene, répondant à la formule R_{b}R_{c}C=CX¹X², où
: X¹ = F ou Cl
X² = H, F ou Cl
chacun de R_{b} et R_{c} représente, indépendamment :
   - H, Cl, F ; ou
   - un groupe alkyle, de préférence chloré et /ou fluoré, plus avantageusement perchloré ou perfluoré.

Les inventeurs ont par ailleurs mis en évidence que la polymérisation en phase dispersée des composés vinyliques halogénés précités peut être obtenue, de façon plus générale, avec des stabilisants réactifs comportant un groupe thiocarbonylthio -S(C=S)-analogues à ceux de la présente invention, mais qui contiennent une chaîne polymère autre qu'une chaîne comprenant des unités monomères (N-vinyl lactame), ou bien une chaîne comprenant moins de 50%, voire moins de 30% d' unités monomères (N-vinyl lactame).

Plus précisément, les inventeurs ont mis en évidence un procédé intéressant de polymérisation en phase dispersée de composés vinyliques halogénés, répondant à la formule R_{b}R_{c}C=CX¹X², où :
X¹ = F ou Cl
X² = H, F ou Cl
chacun de R_{b} et R_{c} represente, indépendamment :
   - H, Cl, F ; ou
   - un groupe alkyle, de préférence chloré et /ou fluoré, plus avantageusement perchloré ou perfluoré,
où ces monomères sont introduits au sein d'une phase aqueuse, en général à l'état dispersé, ensemble avec
- au moins une source de radicaux libres ; et
- au moins un stabilisant réactif dit ci-après « SR2 » qui comprend une chaîne polymère et un groupe thiocarbonylthio -S(C=S)-, cette chaîne polymère comprenant des unités monomères non (N-vinyl lactame) de préférence choisies parmi :
   - les monomères hydrophiles de type acrylate, comme par exemple l'acide acrylique et ses sels tel que acrylate de sodium ainsi que les esters de l'acide acrylique soluble dans l'eau, comme par exemple l'acrylate de 2-hydroxy ethyl ou les acrylates d'oligo ou polyéthylène glycol.
      les monomères hydrophiles de type acrylamido comme par exemple l'acrylamide, dimethyl acrylamide, 2-acrylamido-2-methyl-1-propane sulfonique acide et ses sels, acrylamido propyl trimethyl ammonium chloride (APTAC), diméthyl aminopropyl acrylamide, NN-diethyl acrylamide, N-isopropyl acrylamide, N-morpholine acrylamide, N-hydroxy ethyl acrylamide.
   - les monomères hydrophiles de type methacrylate par exemple l'acide methacrylique et ses sels tel que le methacrylate de sodium ainsi que les methacrylate d'oligo ou polyéthylène glycol, 3-[N-(3-methacrylate de propyl)-N,N-dimethyl]ammoniopropane sulfonate), hydroxyethyl methacrylate.
   - les monomères hydrophiles de type methacrylamido comme par exemple le methacrylamide, 3-[N-(3-methacrylamidopropyl)-N,N-dimethyl]ammoniopropane sulfonate)) (SPP), [(3-methacrylamidopropyl)-N,N-trimethyl ammonium chloride (MAPTAC).
   - les monomères hydrophiles de type vinylique par exemple acide vinyl phosphonique, vinyl sulphonate de sodium, 2-vinyl pyridine, 4-vinyl pyridine, et ses versions quaternisées et imidazole de vinyle
   - les monomères hydrophiles de type allylique par exemple le chlorure de diallyldiméthylammonium (DADMAC), le diallyl de dimethylammoniummethyl phosphonate (DALP)
- éventuellement moins de 50%, voire moins de 30% d'unités monomères N-vinyl lactame.

Avec les monomères halogénés précités, un des avantages du procédé de l'invention se révèle particulièrement intéressant, à savoir la possibilité de substituer les tensioactifs nécessaires en polymérisation en phase dispersée par les agents réactifs de l'invention. En effet, classiquement, la polymérisation en phase dispersée de monomères halogénés du type précité implique la mise en oeuvre de tensioactifs halogénés, le plus souvent fluorés, tels que les fluoroamphipiles du type des sels d'ammonium de l'acide perfluorooctanesulfonique (PFOS), de l'acide perfluorooctanoique (PFOA) ou de l'acide perfluorononanoique (PFNA) dont l'utilisation est de plus en plus controversée, du fait de leur profil toxicologique (toxicité associée à une rémanence).

Ces monomères halogénés peuvent typiquement être mis en oeuvre dans l'étape (E1) telle que décrite dans la présente description, en substituant le stabilisant réactif N-vinyl lactame par un stabilisant réactif de type « SR2 » tel que défini ci-dessus.

L'invention permet de fournir des latex fluorés où les tensioactifs de ce type sont substitués au moins en partie, de préférence en totalité, par des stabilisants réactifs selon l'invention. Dans ce cadre, les stabilisants réactifs employés peuvent être halogénés ou fluorés (par exemple, on peut utiliser des stabilisants réactifs porteurs de groupes fluorés ou chlorés) mais contrairement aux procédés usuels de polymérisation en phase dispersée, le procédé de l'invention, qui emploie des stabilisants *réactifs*, conduit à une immobilisation des stabilisants sur les particules de polymères, et ne conduit donc pas aux effets de relargage de tensioactifs qui peuvent être observés avec les latex fluorés usuels. L'invention autorise donc dans ce cadre l'emploi de stabilisants fluorés avec des impacts réduits en termes de toxicité.

Par ailleurs, le procédé de l'invention permet d'accéder à une polymérisation contrôlée des monomères précités particulièrement efficace et inédite des monomères halogénés précités.

Les monomères halogénés utilisables dans l'étape (E1) selon ce second mode de réalisation incluent par exemple le chlorure de vinyle H2C=CHCl (VC), le chlorure de vinydilène H2C=CCl2 (VC2), le fluorure de vinyle (VF), le fluorure de vinylidène (VDF), l'hexafluoropropène (HFP), le 3,3,3-trifluoropropene (TFP), le tétrafluoroéthylène (TFE), le chlorotrifluoroéthylène (CTFE), ou bien encore les perfluorovinylether (PFVE), comme le perfluorométhylvinylether (PFMVE). Ces monomères peuvent être homopolymérisés ou copolymérisés dans l'étape (E1), entre eux, ou avec d'autres monomères éthyléniquement insaturés, halogénés ou non (typiquement moins de 50% en mole de monomère non halogéné, le cas échéant).

### Les dispersions de polymères synthétisés selon l'invention

Quelle que soit la nature des monomères employés, l'étape (E1) permet d'accéder à des dispersions de polymère. Typiquement, il s'agit un latex comprenant des particules dispersées formées de polymères séquencés issus de la polymérisation radicalaire contrôlée tels qu'obtenus à l'issue de l'étape (E1) qui contiennent des particules dispersées formées de polymères séquencés issus de la polymérisation radicalaire contrôlée conduite dans cette étape, chacun de ces polymères à bloc comprenant :
- un premier bloc, généralement hydrophile, ou éventuellement amphiphile, correspondant à la chaîne polymère à base d'unités de poly(N vinyl lactame) des stabilisants réactifs employés dans l'étape (E1) ; et
- lié de façon covalente à ce premier bloc, un deuxième bloc, hydrophobe, comprenant une chaîne polymère résultant de la polymérisation des monomères éthyléniquement insaturés employés dans l'étape (E1), généralement terminée par le groupe réactif thiocarbonylthio (xanthate par exemple) initialement présent sur le stabilisant réactif employé dans l'étape (E1)

Compte tenu des conditions de mise en oeuvre de l'étape (E1), les polymères à bloc issus de cette étape s'agencent, schématiquement, au sein de chaque particule pour former un « coeur » de particule à base des blocs hydrophobes et une « coquille » extérieure à base des chaînes polymères de poly(N vinyl lactame), liées de façon covalente aux chaînes du coeur.

Les latex ainsi obtenus présentent l'avantage d'être stables sans nécessiter la présence d'agents tensioactifs. Bien que la mise en oeuvre d'un tensioactif additionnel ne soit pas exclue, il est le plus souvent intéressant qu'aucun tensioactif additionnel ne soit mis en oeuvre dans l'étape (E1) ou postérieurement à celle-ci. ce qui permet d'obtenir des latex exempts de tensioactifs. L'immobilisation de l'agent stabilisant sur la particule par greffage covalent, intrinsèque à la mise en oeuvre de l'étape (E1), participe à la stabilisation du latex, en inhibant les phénomènes de désorption qu'on observe quand on emploie des tensioactifs usuels au lieu des agents stabilisants réactifs de l'invention.

Par ailleurs, la mise en oeuvre de l'étape (E1) permet d'obtenir une chimie de surface contrôlée à la surface des particules du latex, modulable en fonction de la nature de la chaîne polymère des stabilisants réactifs sur laquelle des groupes réactifs peuvent être introduits :
- avant l'étape (E1) :notamment en introduisant des monomères, fonctionnalisés ou non, dans l'étape (E0), par exemple en copolymérisant dans l'étape (E0) les N-vinyl lactame avec d'autres monomères, comme par exemple choisis parmi les acrylamides, N,N-dimethyl acrylamides, 2-acrylamido-2-methylpropanesulfonic acid (AMPS), (acrylamido propyl trimethyl ammonium chloride (APTAC), ou acide acrylique ; et/ou
- après l'étape (E1): par exemple par réaction de greffage *via* des groupes présents sur les chaînes polymères poly(N vinyl lactame) immobilisées à la surface des particules du latex.

L'utilisation de la polymérisation radicalaire contrôlée dans l'étape (E1) permet par ailleurs un contrôle extrêmement fin de la masse moléculaire en nombre Mn des polymères synthétisés, et ce de façon très simple et directe. Partant, l'étape (E1) permet un contrôle fin très aisé de la taille des particules formées, ce qui peut être utilisé notamment pour optimiser la stabilité colloïdale. De plus, on obtient des particules de polymères qui tendent, de la même façon à avoir des diamètres homogènes et resserrés autour d'une valeur moyenne.

Les polymères obtenus selon l'étape (E1) sous la forme de particules présentent un caractère vivant et peuvent donc, dans l'absolu, être utilisés à titre de polymères vivants pour la synthèse ultérieure de copolymères séquencés sur lesquels un troisième bloc sera greffé. Dans ce cadre, les polymères obtenus dans l'étape (E1) peuvent être utilisés à titre d'agents de contrôle dans une étape de polymérisation postérieure à l'étape (E1).

Le plus souvent, toutefois, ces polymères sont employés à l'état de latex. Dans ce cas, il peut être souhaitable de désactiver l'extrémité réactive des polymères obtenu à l'issue de l'étape (E1). Cette désactivation peut être opérée après l'étape (E1), ou bien elle peut terminer cette étape (par exemple, elle peut être mise en oeuvre lorsque la taille la masse moléculaire souhaitée ou le diamètre des particules du latex attendu est atteint). Dans ce cadre, le procédé comprend alors, après l'étape (E1), une étape (E2) de traitement chimique de l'extrémité de chaîne. Par exemple, lorsque le stabilisant réactif employé dans l'étape (E1) est un xanthate, l'extrémité réactive xanthate obtenue sur le polymère peut être désactivée pour priver le polymère de son caractère vivant, par exemple par action de peroxyde d'hydrogène ou par action d'un organoperoxyde ou bien encore d'ozone, qui oxyde l'extrémité réactive xanthate en diverses espèces oxydées (notamment de type thioester -S(C=O)- et -SO₃H).

Selon un mode de réalisation spécifique, la chaîne polymère du stabilisant réactif employé dans l'étape (E1) est à base de monomères qui sont capables d'assurer la stabilité colloïdale des particules de latex dans certaines conditions mais qui perdent cette propriété (ou bien la voient réduite) dans d'autres conditions (par exemple sous l'effet d'un changement de température, de pH ou de salinité), ce par quoi le latex obtenu est sensible à un stimulus où on obtient une floculation du latex. A titre d'exemple, on obtient un effet de ce type pour des stabilisants réactifs de type poly(N-vinylcaprolactame) pour lesquels on obtient une stabilité colloïdale à basse température et une floculation au dessus d'une température seuil.

Les latex obtenus à l'issue de l'étape (E1) peuvent être employés dans un très grand nombre d'applications, notamment pour la réalisation de peintures, revêtements et adhésifs, pour la préparation de matériaux de construction, ou bien encore dans des compositions cosmétiques ou de soin corporel, dans des formulations phytosanitaires ou destinées au domaine de l'agriculture ou bien encore dans des fluides pour l'extraction pétrolière.

Selon un mode de réalisation particulier, la dispersion de polymère issue de l'étape (E1) qui comprend des polymères séquencés issus de la polymérisation radicalaire contrôlée tels qu'obtenus à l'issue de l'étape (E1), organisés sous forme de micelles, utilisables à titre de site de polymérisation pour des polymérisations radicalaires contrôlées.

L'invention et ses avantages seront encore davantage illustrés par les exemples de mise en oeuvre donnés ci-après.

### Exemples

### Exemple 1

### Préparation d'un stabilisant réactif NVP-Xa

Dans un ballon de 250ml à température ambiante (20°C), on a introduit 120 g de N-vinyl pyrrolidone, 60 g d'eau distillée, 9 g de O-ethyl-S-(1-methoxycarbonyl ethyl) xanthate de formule (CH₃CH(CO₂CH₃))S(C=S)OEt et 2,1 g d'une solution d'hydroperoxyde de tertbutyle (70 % massique dans l'eau).

Le mélange réactionnel a été dégazé par bullage d'azote pur pendant 30 minutes. Ensuite, 2,1g de sulfite de sodium a été ajouté en une fois sous courant d'azote.

On a laissé le milieu réactionnel sous agitation pendant 24 heures à température ambiante.

A l'issue de la réaction, une conversion de 95 % a été déterminée par RMN ¹H. La présence de l'extrémité xanthate est également observée en RMN ¹H.

### Exemple 2

### Dexanthation du stabilisant réactif NVP-Xa ------> NVP

Dans un ballon de 100ml à température ambiante (20°C) on a introduit 30g de la solution de NVP-Xa. On a chauffé le mélange à 90°C pendant 5h sous agitation. L'élimination du bout de chaîne xanthate a été confirmé par une analyse par spectrophotométrie UV/VIS. La disparition du pic a 262nm qui est caractéristique de CS(C=S)OEt met en évidence une dexanthation réussie.

### Exemple 3

### Polymérisation en émulsion de l'acétate de vinyle en présence de NVP-Xa

Dans un ballon de 250ml à température ambiante (20°C), on a introduit 10 g d'acétate de vinyle 90 g d'eau distillée, 0,42 g de NVP-Xa (Exemple 1) et 0,14 g d'une solution d'hydroperoxyde de tertbutyle (70 % massique dans l'eau).

Le mélange réactionnel a été dégazé par un léger bullage d'azote pur pendant 5 minutes. Ensuite, 0,1g de sulfite de sodium a été ajouté en une fois sous courant d'azote.

On a laissé le milieu réactionnel sous agitation pendant 12 heures à température ambiante.

A l'issue de la réaction, une conversion de 89 % a été déterminée par gravimétrie

Une analyse en diffusion de la lumière statique (malvern zetasizer) fournit les valeurs en diamètre de particule Dz de 123 nm et une polydispersité de particules de 0.01.

### Exemple 4 : Polymérisation en émulsion de l'acétate de vinyle en présence de NVP-Xa

Dans un ballon de 250ml à température ambiante (20°C), on a introduit 10 g d'acétate de vinyle 90 g d'eau distillée, 4,2 g de NVP-Xa (Exemple 1) et 0,14 g d'une solution d'hydroperoxyde de tertbutyle (70 % massique dans l'eau).

Le mélange réactionnel a été dégazé par une léger bullage d'azote pur pendant 5 mins minutes. Ensuite, 0,1g de sulfite de sodium a été ajouté en une fois sous courant d'azote.

On a laissé le milieu réactionnel sous agitation pendant 12 heures à température ambiante.

A l'issue de la réaction, une conversion de 81 % a été déterminée par gravimétrie.

Une analyse en diffusion de la lumière statique (malvern zetasizer) fournit les valeurs en diamètre de particule Dz de 36 nm et une polydispersité de particules de 0.2.

### Exemple 5 : Polymérisation en émulsion de l'acétate de vinyle en présence de NVP-Xa

Dans un ballon de 250ml à température ambiante (20°C), on a introduit 37,04 g d'acétate de vinyle 90 g d'eau distillée, 1,56 g de NVP-Xa (Exemple 1) et 0,53 g d'une solution d'hydroperoxyde de tertbutyle (70 % massique dans l'eau).

Le mélange réactionnel a été dégazé par un léger bullage d'azote pur pendant 5 minutes. Ensuite, 0,37 g de sulfite de sodium a été ajouté en une fois sous courant d'azote.

On a laissé le milieu réactionnel sous agitation pendant 12 heures à température ambiante. A la fin de la réaction on a obtenue un latex stable sans coagulum.

A l'issue de la réaction, une conversion de 97 % a été déterminée par gravimétrie.

### Exemple 6 : Polymérisation en émulsion de l'acétate de vinyle en présence de NVP Dexanthaté (exemple 2)

Dans un ballon de 250ml à température ambiante (20°C), on a introduit 37,04 g d'acétate de vinyle 90 g d'eau distillée, 1,56 g de NVP (Exemple 2) et 0,53 g d'une solution d'hydroperoxyde de tertbutyle (70 % massique dans l'eau).

Le mélange réactionnel a été dégazé par un léger bullage d'azote pur pendant 5 minutes. Ensuite, 0,37 g de sulfite de sodium a été ajouté en une fois sous courant d'azote.

On a laissé le milieu réactionnel sous agitation.

Apres 180 minutes on observe que le latex a totalement floculé.

### Exemple 7 : Polymérisation en émulsion de chlorure de vinylidene (VDC) en présence de NVP-Xa

Dans un ballon de 250ml à température ambiante (20°C), on a introduit 10 g de VDC 59 g d'eau distillée, 0,43 g de NVP-Xa (Exemple 1) et 0,27 g d'une solution d'hydroperoxyde de tertbutyle (70 % massique dans l'eau).

Le mélange réactionnel a été refroidi à 10°C et dégazé par un léger bullage d'azote pur pendant 5 minutes sous agitation (barreau magnétique). Ensuite, 0,26g de sulfite de sodium a été ajouté en une fois sous courant d'azote.

On a laissé le milieu réactionnel sous agitation pendant 3h heures à 10°C (+/- 5°C).

A l'issue de la réaction, une conversion de 87 % a été déterminée par gravimétrie.

Une analyse en diffusion de la lumière statique (Malvern Zetasizer) fournit les valeurs en diamètre de particule Dz de 222 nm et une polydispersité de particules de 0.03.

### Exemple 8: Polymérisation en émulsion de chlorure de vinylidene (VDC) en présence de NVP-Xa

Dans un ballon de 250ml à température ambiante (20°C), on a introduit 10 g de VDC 59 g d'eau distillée, 1,31g de NVP-Xa (Exemple 1) et 0,27 g d'une solution d'hydroperoxyde de tertbutyle (70 % massique dans l'eau).

Le mélange réactionnel a été refroidi à 10°C et dégazé par un léger bullage d'azote pur pendant 5 minutes sous agitation (barreau magnétique). Ensuite, 0,26g de sulfite de sodium a été ajouté en une fois sous courant d'azote.

On a laissé le milieu réactionnel sous agitation pendant 3h heures à 10°C (+/- 5°C).

A l'issue de la réaction, une conversion de 87 % a été déterminée par gravimétrie.

Une analyse en diffusion de la lumière statique (Malvern Zetasizer) fournit les valeurs en diamètre de particule Dz de 192 nm et une polydispersité de particules de 0.01

### Exemple 9: Polymérisation en émulsion de chlorure de vinylidene (VDC) en présence de Polyacrylamide-Xa

Dans un ballon de 250ml à température ambiante (20°C), on a introduit 10 g de VDC 59 g d'eau distillée, 0,47 g de Polyacrylamide-Xa (Xa=Rhodixan A1, Mn=3000 g/mol, synthétisé selon Taton et al. Macromol. Rapid Commun. 2001, 22, 18, 1497) et 0,26 g d'une solution d'hydroperoxyde de tertbutyle (70 % massique dans l'eau).

Le mélange réactionnel a été refroidi à 10°C et dégazé par un léger bullage d'azote pur pendant 5 minutes sous agitation (barreau magnétique). Ensuite, 0,26g de sulfite de sodium a été ajouté en une fois sous courant d'azote.

On a laissé le milieu réactionnel sous agitation pendant 3h heures à 10°C (+/- 5°C).

A l'issue de la réaction, une conversion de 91 % a été déterminée par gravimétrie.

Une analyse en diffusion de la lumière statique (Malvern Zetasizer) fournit les valeurs en diamètre de particule Dz de 241 nm et une polydispersité de particules de 0.04.

### Exemple 10 : Polymérisation en émulsion de chlorure de vinylidene (VDC) en présence de Poly(acide acrylique)-Xa

Dans un ballon de 250ml à température ambiante (20°C), on a introduit 10 g de VDC 59 g d'eau distillée, 0,47 g de Polyacide acrylique-Xa (Xa=Rhodixan A1, Mn=3200 g/mol, synthétisé selon Taton et al. Macromol. Rapid Commun. 2001, 22, 18, 1497) et 0,26 g d'une solution d'hydroperoxyde de tertbutyle (70 % massique dans l'eau).

Le mélange réactionnel a été refroidi à 10°C et dégazé par un léger bullage d'azote pur pendant 5 minutes sous agitation (barreau magnétique). Ensuite, 0,26 g de sulfite de sodium a été ajouté en une fois sous courant d'azote.

On a laissé le milieu réactionnel sous agitation pendant 3h heures à 10°C (+/- 5°C).

A l'issue de la réaction, une conversion de 89 % a été déterminée par gravimétrie.

Une analyse en diffusion de la lumière statique (Malvern Zetasizer) fournit les valeurs en diamètre de particule Dz de 195 nm et une polydispersité de particules de 0.03.

## Revendications

1. Procédé de préparation d'un polymère comprenant une étape (E1) de polymérisation en phase dispersée en présence d'un stabilisant réactif, dans laquelle on met en présence au sein d'une phase aqueuse :
- au moins un monomère éthyléniquement insaturé;
- au moins une source de radicaux libres ; et
- un stabilisant réactif comprenant une chaîne polymère incluant des unités monomères (N-vinyl lactame) et un groupe thiocarbonylthio -S(C=S)-

2. Procédé selon la revendication 1 où dans l'étape (E1), le monomère éthyléniquement insaturé est à l'état dispersé.

3. Procédé selon la revendication 1 où l'étape (E1) est conduite à une température inférieure à 40°C, de préférence inférieure ou égale à 30°C.

4. Procédé selon la revendication 3, où la source de radicaux libre employée dans l'étape (E1) est un initiateur de polymérisation redox.

5. Procédé selon la revendication 4, où l'initiateur redox comprend de l'hydroperoxyde de tertbutyle (t-BuOOH) à titre d'agent oxydant, associé à un agent réducteur choisi parmi l'acide ascorbique ou le sulfite de sodium.

6. Procédé selon l'une des revendications 1 à 4, où le stabilisant réactif employé dans l'étape (E1), des monomères éthyléniquement insaturés répondant en général à la formule suivante : 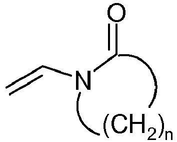 où n est un entier allant de 2 à 6.

7. Procédé selon la revendication 6, où le stabilisant réactif employé dans l'étape (E1), des monomères N-vinyl pyrrolidone, N-vinyl pipéridone ou N-vinyl caprolactame.

8. Procédé selon la revendication 1 à 7, où le groupement thiocarbonylthio présent sur le stabilisant réactif est un xanthate ou un dithiocarbamate, de préférence un xanthate.

9. Procédé selon l'une des revendications 1 à 8, où les monomères employés dans l'étape (E1) sont des monomères esters de vinyle de formule CH₂=CH-O-(C=O)-Rₐ, où Rₐ représente :
• un groupe alkyle, acyle, aryle, aralkyle, alcène ou alcyne éventuellement substitué, de préférence un groupe alkyle
• un cycle carboné ou un hétérocycle, saturé ou non, aromatique éventuellement substitué.

10. Procédé selon l'une des revendications 1 à 8, où les monomères employés dans l'étape (E1) comprennent des composés vinyliques halogénés, tels que des chlorures ou fluorure de vinyle ou de vinylidene, répondant à la formule R_{b}R_{c}C=CX¹X², où :
X¹ = F ou Cl
X² = H, F ou Cl
chacun de R_{b} et R_{c} represente, indépendamment :
- H, Cl, F ; ou
- un groupe alkyle, de préférence chloré et /ou fluoré, plus avantageusement perchloré ou perfluoré.

11. Dispersions de polymères susceptibles d'être obtenu selon le procédé de l'une des revendications 1 à 9.

12. Dispersion selon la revendication 11, qui est un latex comprenant des particules dispersées formées de polymères séquencés issus de la polymérisation radicalaire contrôlée tels qu'obtenus à l'issue de l'étape (E1) de la revendication 1.

13. Utilisation d'une dispersion selon la revendication 12, pour la réalisation de peintures, revêtements, adhésifs, pour la préparation de matériaux de construction, dans des compositions cosmétiques ou de soin corporel, dans des formulations phytosanitaires ou destinées au domaine de l'agriculture ou dans des fluides pour l'extraction pétrolière.

14. Dispersion selon la revendication 11, qui comprend des polymères séquencés issus de la polymérisation radicalaire contrôlée tels qu'obtenus à l'issue de l'étape (E1) de la revendication 1, organisés sous forme de micelles, utilisables à titre de site de polymérisation pour des polymérisations radicalaires contrôlées.

## Patentansprüche

1. Verfahren zur Herstellung eines Polymers, das einen Schritt (E1) der Polymerisation in dispergierter Phase in Gegenwart eines reaktiven Stabilisators umfasst, wobei man in einer wässrigen Phase Folgendes in Kontakt bringt:
- mindestens ein ethylenisch ungesättigtes Monomer;
- mindestens eine Quelle für freie Radikale; und
- einen reaktiven Stabilisator, der eine Polymerkette umfasst, die N-(Vinyllactam)-Monomereinheiten und eine Thiocarbonylthiogruppe -S(C=S)- umfasst.

2. Verfahren nach Anspruch 1, wobei in Schritt (E1) das ethylenisch ungesättigte Monomer im dispergierten Zustand vorliegt.

3. Verfahren nach Anspruch 1, wobei Schritt (E1) bei einer Temperatur unter 40°C, vorzugsweise unter oder gleich 30°C durchgeführt wird.

4. Verfahren nach Anspruch 3, wobei die Quelle für die in Schritt (E1) verwendeten freien Radikale ein Redoxpolymerisationsstarter ist.

5. Verfahren nach Anspruch 4, wobei der Redoxstarter tert-Butylhydroperoxid (t-BuOOH) als Oxidationsmittel in Verbindung mit einem Reduktionsmittel, das aus Ascorbinsäure und Natriumsulfit ausgewählt ist, umfasst.

6. Verfahren nach einem der Ansprüche 1 bis 4, wobei der in Schritt (E1) eingesetzte reaktive Stabilisator, ethylenisch ungesättigte Monomere mit der folgenden allgemeinen Formel: 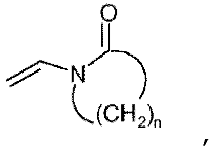 wobei n eine ganze Zahl von 2 bis 6 ist.

7. Verfahren nach Anspruch 6, wobei der in Schritt (E1) eingesetzte reaktive Stabilisator, N-Vinylpyrrolidon-, N-Vinylpiperidon- oder N-Vinylcaprolactam-Monomere.

8. Verfahren nach Anspruch 1 bis 7, wobei die an dem reaktiven Stabilisator vorhandene Thiocarbonylthiogruppe ein Xanthat oder ein Dithiocarbamat, vorzugsweise ein Xanthat ist.

9. Verfahren nach einem der Ansprüche 1 bis 8, wobei die in Schritt (E1) eingesetzten Monomere Vinylester-Monomere der Formel CH2=CH-O-(C=O)-Rₐ sind, wobei Rₐ für Folgendes steht:
- eine gegebenenfalls substituierte Alkyl-, Acyl-, Aryl-, Aralkyl-, Alken- oder Alkingruppe, vorzugsweise eine Alkylgruppe
- einen gesättigten oder ungesättigten, aromatischen, gegebenenfalls substituierten Carbocyclus oder Heterocyclus.

10. Verfahren nach einem der Ansprüche 1 bis 8, wobei die in Schritt (E1) eingesetzten Monomere halogenierte Vinylverbindungen, wie Vinyl- oder Vinylidenchloride oder -fluorid, mit der Formel R_{b}R_{c}C=CX¹X² umfassen, wobei:
X¹ = F oder Cl
X² = H, F oder Cl
R_{b} und R_{c} jeweils unabhängig Folgendes darstellen:
- H, Cl, F oder
- eine Alkylgruppe, die vorzugsweise chloriert und/oder fluoriert, noch vorteilhafter perchloriert oder perfluoriert ist.

11. Dispersionen von Polymeren, die gemäß dem Verfahren nach einem der Ansprüche 1 bis 9 erhalten werden können.

12. Dispersion nach Anspruch 11, die ein Latex ist, der dispergierte Teilchen umfasst, die von Blockpolymeren gebildet werden, die aus der kontrollierten radikalischen Polymerisation herrühren, wie sie am Ende von Schritt (E1) von Anspruch 1 erhalten werden.

13. Verwendung einer Dispersion nach Anspruch 12 zur Herstellung von Anstreichmitteln, Beschichtungen, Klebstoffen für die Herstellung von Bauwerkstoffen, in kosmetischen oder Körperpflegezusammensetzungen, in Pflanzenschutzformulierungen oder für das Gebiet der Landwirtschaft bestimmten Formulierungen oder in Erdölförderungsfluiden.

14. Dispersion nach Anspruch 11, die Blockpolymere umfasst, die aus der kontrollierten radikalischen Polymerisation herrühren, wie sie am Ende von Schritt (E1) von Anspruch 1 erhalten werden, die in Form von Mizellen organisiert sind und als Polymerisationsstelle für kontrollierte radikalische Polymerisationen verwendbar sind.

## Claims

1. Process for preparing a polymer, comprising a step (E1) of dispersed-phase polymerization in the presence of a reactive stabilizer, in which the following are placed in contact in an aqueous phase:
- at least one ethylenically unsaturated monomer;
- at least one source of free radicals; and
- a reactive stabilizer comprising a polymer chain including N-(vinyllactam) monomer units and a thiocarbonylthio group -S(C=S)-.

2. Process according to Claim 1, in which, in step (E1), the ethylenically unsaturated monomer is in dispersed form.

3. Process according to Claim 1, in which step (E1) is performed at a temperature below 40°C, preferably less than or equal to 30°C.

4. Process according to Claim 3, in which the source of free radicals used in step (E1) is a redox polymerization initiator.

5. Process according to Claim 4, in which the redox initiator comprises tert-butyl hydroperoxide (t-BuOOH) as oxidizing agent, combined with a reducing agent chosen from ascorbic acid and sodium sulfite.

6. Process according to one of Claims 1 to 4, in which the reactive stabilizer used in step (E1), ethylenically unsaturated monomers generally corresponding to the following formula: 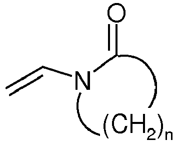 in which n is an integer ranging from 2 to 6.

7. Process according to Claim 6, in which the reactive stabilizer used in step (E1), monomers N-vinylpyrrolidone, N-vinylpiperidone or N-vinyl-caprolactam.

8. Process according to Claim 1 to 7, in which the thiocarbonylthio group present on the reactive stabilizer is a xanthate or a dithiocarbamate, preferably a xanthate.

9. Process according to one of Claims 1 to 8, in which the monomers used in step (E1) are vinyl ester monomers of formula CH2=CH-O-(C=O)-Rₐ, in which Rₐ represents:
• an optionally substituted alkyl, acyl, aryl, aralkyl, alkene or alkyne group, preferably an alkyl group,
• a saturated or unsaturated, aromatic, optionally substituted carbocycle or heterocycle.

10. Process according to one of Claims 1 to 8, in which the monomers used in step (E1) comprise halogenated vinyl compounds, such as vinyl or vinylidene chlorides or fluoride, corresponding to the formula R_{b}R_{c}C =CX¹X², in which:
X¹ - F or Cl
X² - H, F or Cl
each one from among R_{b} and R_{c} represents, independently:
- H, Cl, F; or
- an alkyl group, preferably chlorinated and/or fluorinated, more advantageously perchlorinated or perfluorinated.

11. Dispersions of polymers which may be obtained according to the process of one of Claims 1 to 9.

12. Dispersion according to Claim 11, which is a latex comprising dispersed particles formed from block polymers derived from the controlled radical polymerization, as obtained after step (E1) of Claim 1.

13. Use of a dispersion according to Claim 12, for producing paints, coatings and adhesives, for preparing construction materials, in cosmetic or body care compositions, in plant protection formulations or for the agricultural sector, or in petroleum extraction fluids.

14. Dispersion according to Claim 11, which comprises block polymers derived from the controlled radical polymerization, as obtained after step (E1) of Claim 1, organized in the form of micelles, which may be used as a site of polymerization for controlled radical polymerizations.
